# EUROPEAN PATENT SPECIFICATION

(11) **EP 3 903 620 B1**
(45) Date of publication and mention of the grant of the patent: **30.03.2022**
(21) Application number: 21158311.7
(22) Date of filing: 21.02.2021
(51) Int. Cl.: A43B 7/14

(54) **SHOES FOR MASSAGING THE INNER SIDE OF HALLUX**
SCHUHE ZUM MASSIEREN DER INNENSEITE DES HALLUX
CHAUSSURES DE MASSAGE DE LA FACE INTÉRIEURE DE L'HALLUX

(30) Priority: 28.04.2020 CN 202020677581 U
(43) Date of publication of application: 03.11.2021
(73) Proprietor: Guangdong Breath Walker Health Technology Co., Ltd., Dongguan City, Guangdong Province (CN)
(72) Inventor: Wu, Xingliang, Dongguan City, Guangdong Province (CN); Zhou, Yuyuan, Dongguan City, Guangdong Province (CN); Liu, Kanglong, Dongguan City, Guangdong Province (CN)
(74) Representative: Cabinet Chaillot

(56) References cited:
- WO-A1-2016/166749
- KR-U- 20110 010 287
- KR-Y1- 200 304 935

## Description

### FIELD OF THE INVENTION

The present invention relates to shoes, and more particularly to the shoes for massaging the inner side of hallux.

### BACKGROUND OF THE INVENTION

At present, massage can be divided into head massage, neck massage, waist massage, back massage, buttock massage, foot massage, etc. Among these massages, foot massage plays an important role in people's health and regular medical healthcare, but most foot massages are focused on the soles of the feet, while the massage between the toes is ignored.

In addition, hallux valgus is one of the most common diseases of the foot, and it is also the most common disease of the hallux (or big toe). Hallux valgus is primarily represented by the position of the big toe deviating outward to have an angle greater than 15° between the proximal phalanx of hallux and the first metatarsal. The most common problems caused by hallux valgus include pain and difficulty in wearing shoes. In severe cases, hallux valgus can also cause arthritis and degenerative changes in the hallux metatarsophalangeal joint; and it can also induce deformity and dislocation of the second toe.

However, the existing surgical treatments for correcting hallux valgus has the drawbacks of complicated operation, trauma of patients' body, and long recovery time, which will cause lots of inconvenience to people's body, mind, and life. Existing functional shoes cannot prevent and correct the condition of hallux valgus and will aggravate the situation of hallux valgus and affect the relief of hallux valgus.

WO 2016/166749 A1 disclose a footwear according to prior art.

### SUMMARY OF THE INVENTION

It is a primary objective to provide a pair of shoes for massaging the inner side of a hallux in order to solve the problems that the traditional massage shoes cannot massage the inner side of the hallux or prevent and correct hallux valgus, and the existing surgical correction procedures for the treatment of hallux valgus is complicated.

To achieve the aforementioned and other objectives, the present invention discloses a shoe for massaging the inner side of hallux, comprising a sole, a vamp mounted on the sole, and an elastic correction band, and both ends of the elastic correction band are installed to an inner side of the vamp and a top side of the sole; the elastic correction band has a massage area defined in the middle of an outer surface on a side of the hallux, and a plurality of massage bumps formed on the massage area; the elastic correction band further has a fixed part disposed separately on both sides of the massage area, and the fixed part at the upper end of the elastic correction band is fixedly installed to an inner side of the vamp, and the fixed part at the lower end of the elastic correction band fixedly installed to a top side of the sole.

Further, the sole comprises an outsole and a Laban insole installed on a top side of the outsole, and the Laban insole has a via formed thereon, and a bottom end of the elastic correction band is passed through the via and then folded and fixed to a back side of the Laban insole.

In summation, the elastic correction band of the present invention not just can massage the inner side of hallux to achieve the effect of relieving migraine only, but also can provide the effect of correcting hallux valgus while providing a certain buffering effect by the elasticity of the elastic correction band to avoid foot pain caused by forced correction and prevent increased valgus. The elastic correction band of this invention has the features of simple structure, convenient installation, high practicality and strong promotional effect.

### BRIEF DESCRIPTION OF THE DRAWINGS

FIG. 1 is a schematic view of a massage shoe for the inner side of hallux in accordance with the present invention;
FIG. 2 is a cross-sectional view of Section A-A of FIG. 1; and
FIG. 3 is a schematic view showing an unfolded structure of an elastic correction band as depicted in FIG. 2.

### DESCRIPTION OF THE PREFERRED EMBODIMENTS

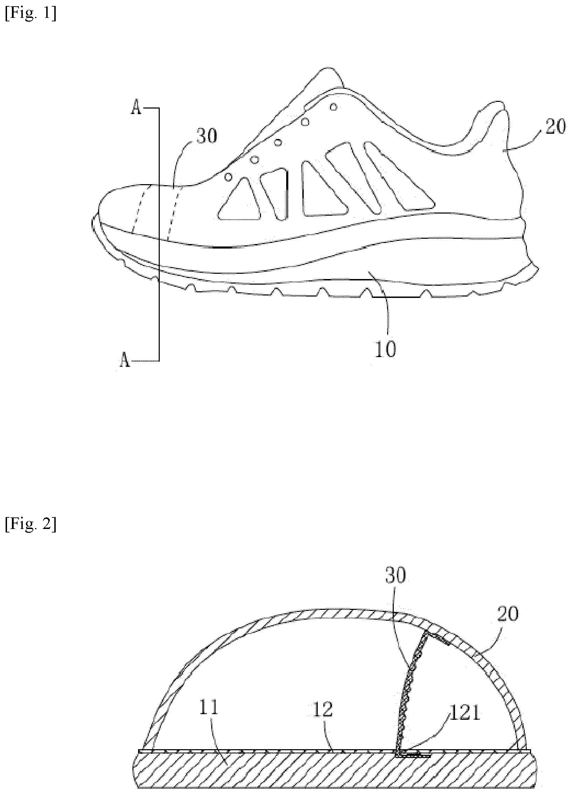

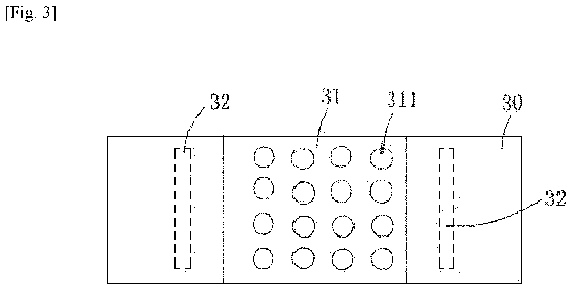

With reference to FIGS. 1 to 3 for a massage shoe for the inner side of hallux in accordance with the present invention, the shoe comprises a sole 10, a vamp 20 mounted on the sole 10, and an elastic correction band 30 with both ends installed to an inner side of the vamp 20 and the top side of the sole 10.

The sole 10 comprises an outsole 11 and a Laban insole 12 disposed on a top side of the outsole 11, and the Laban insole 12 has a via 121 formed thereon. The elastic correction band 30 has a massage area 31 defined at the middle of the elastic correction band 30, and a plurality of massage bumps 311 formed on the massage area 31. The elastic correction band 30 has a fixed part 32 disposed separately on both left and right sides of the massage area 31, and the two fixed parts 32 can be fixed onto a bottom side of the Laban insole 12 and an inner side of the vamp 20 by pasting, sewing or high-temperature heat pressing.

During installation, the elastic correction band 30 is installed between the hallux and the second toe, and the side of the elastic correction band 30 with the massage bumps 311 is configured to be facing the hallux. The fixed part 32 at the top end of the elastic correction band 30 is fixedly installed onto the inner side of the vamp 20, and a bottom end of the elastic correction band 30 is passed through the via 121 of the Laban insole 12 and then folded and fixed onto a bottom side of the Laban insole 12.

In use, the massage area 31 of the elastic correction band 30 can massage the trigeminal nerve acupoints on the inner side of the hallux to achieve the effect of relieving migraine. In addition, the elastic correction band 30 can separate the hallux from the second toe to correct the hallux valgus and provide a certain buffering effect by its elasticity, so as to avoid foot pain caused by forced correction and prevent increased valgus. This invention has high practicality and strong promotional effect.

In summation of the description above, the elastic correction band 30 of the present invention not just can massage the inner side of hallux to achieve the effect of relieving migraine only, but also can provide the effect of correcting hallux valgus while providing a certain buffering effect by the elasticity of the elastic correction band 30 to avoid foot pain caused by forced correction and prevent increased valgus. The elastic correction band 30 of this invention has the features of simple structure, convenient installation, high practicality and strong promotional effect.

### Brief Description of Numerals Used in the Drawings

10: sole; 11: outsole; 12: Laban insole; 121: via; 20: vamp; 30: elastic correction band; 31: massage area; 32: fixed part; 311: massage bumps.

## Claims

1. A shoe for massaging the inner side of hallux, comprising: a sole (10), a vamp (20) mounted on the sole (10);
an elastic correction band (30) with both ends installed to an inner side of the vamp (20) and a top side of the sole (10) respectively; the elastic correction band (30) comprising a massage area (31) defined in the middle of an outer surface of a side of the hallux, and a plurality of massage bumps (311) disposed on the massage area (31); the elastic correction band (30) comprising a fixed part (32) disposed separately on both sides of the massage area (31), and the fixed part (32) at an upper end of the elastic correction band (30) being fixedly installed onto an inner side of the vamp (20), and the fixed part (32) at a lower end of the elastic correction band (30) being fixedly installed onto a top side of the sole (10).

## Patentansprüche

1. - Schuh zum Massieren der Innenseite des Hallux, umfassend: eine Sohle (10), ein Oberleder (20), das auf die Sohle (10) montiert ist,
ein elastisches Korrekturband (30), von dem beide Enden an einer Innenseite des Oberleders (20) bzw. an einer Oberseite der Sohle (10) installiert sind; wobei das elastische Korrekturband (30) einen Massagebereich (31) umfasst, der in der Mitte einer Außenfläche einer Seite des Hallux definiert ist, und eine Vielzahl von Massageerhebungen (311), die auf dem Massagebereich (31) angeordnet sind; wobei das elastische Korrekturband (30) einen festen Teil (32) umfasst, der getrennt auf beiden Seiten des Massagebereichs (31) angeordnet ist, und wobei der feste Teil (32) an einem oberen Ende des elastischen Korrekturbands (30) fest auf einer Innenseite des Oberleders (20) installiert ist, und wobei der feste Teil (32) an einem unteren Ende des elastischen Korrekturbands (30) fest auf einer Oberseite der Sohle (10) installiert ist.

## Revendications

1. - Chaussure pour masser le côté interne de l'hallux, comprenant : une semelle (10), une empeigne (20) montée sur la semelle (10) ;
une bande de correction élastique (30) dont les deux extrémités sont installées sur un côté interne de l'empeigne (20) et un côté supérieur de la semelle (10), respectivement ; la bande de correction élastique (30) comprenant une zone de massage (31) définie au milieu d'une surface externe d'un côté de l'hallux, et une pluralité de bosses de massage (311) disposées sur la zone de massage (31) ; la bande de correction élastique (30) comprenant une partie fixe (32) disposée séparément sur les deux côtés de la zone de massage (31), et la partie fixe (32) à une extrémité supérieure de la bande de correction élastique (30) étant installée de manière fixe sur un côté interne de l'empeigne (20), et la partie fixe (32) à une extrémité inférieure de la bande de correction élastique (30) étant installée de manière fixe sur un côté supérieur de la semelle (10).
